**Europäisches Patentamt**

**European Patent Office**

**Office européen des brevets**

(19)

(11) **EP 1 068 360 B1**

(12) **EUROPÄISCHE PATENTSCHRIFT**

(45) Veröffentlichungstag und Bekanntmachung des Hinweises auf die Patenterteilung:
**16.07.2003 Patentblatt 2003/29**

(21) Anmeldenummer: **99917946.8**

(22) Anmeldetag: **01.04.1999**

(51) Int Cl.$^7$: **C12Q 1/68**

(86) Internationale Anmeldenummer:
**PCT/EP99/02242**

(87) Internationale Veröffentlichungsnummer:
**WO 99/050446 (07.10.1999 Gazette 1999/40)**

(54) **VERFAHREN UND VORRICHTUNG ZUR QUANTIFIZIERUNG VON DNA UND RNA**

METHOD AND DEVICE FOR QUANTIFYING DNA AND RNA

PROCEDE ET DISPOSITIF POUR LA QUANTIFICATION D'ADN ET D'ARN

(84) Benannte Vertragsstaaten:
**CH DE FR GB LI**

(30) Priorität: **01.04.1998 DE 19814682**

(43) Veröffentlichungstag der Anmeldung:
**17.01.2001 Patentblatt 2001/03**

(73) Patentinhaber: **ATTO-TEC GmbH**
**57076 Siegen (DE)**

(72) Erfinder:
• **Wolfrum, Jürgen, Prof.Dr.**
**37124 Rosdorf-Obernjesa (DE)**
• **Sauer, Markus, Dr.**
**69124 Heidelberg (DE)**
• **Han, Kyung-Tae, Dr.**
**71453 Wüstenrot (DE)**

(74) Vertreter: **Köllner & Brunotte**
**Patentanwälte**
**Robert-Bosch-Strasse 7**
**64293 Darmstadt (DE)**

(56) Entgegenhaltungen:
EP-A- 0 819 769         WO-A-95/08646
GB-A- 2 273 772

**Beschreibung**

Die Erfindung betrifft ein Verfahren und eine Vorrichtung zur Quantifizierung von DNA und RNA Sequenzen. Insbesondere betrifft die Erfindung ein Verfahren und eine Vorrichtung zur Detektion einer Amplifizierung einer DNA- und/ oder RNA-Sequenz in einer Probe, insbesondere die Online-Detektion einer Amplifizierung einer DNA- und/ oder RNA-Sequenz in einer Probe.

Der Nachweis von speziellen DNA und RNA Sequenzen, die sich in einer Probe befinden, durch die Versetzung der Probe mit einer komplementären DNA bzw. RNA Sequenz ist ein übliches diagnostisches Verfahren. Für die Bewertung des Ergebnisses ist es erforderlich, daß der Amplifizierungsvorgang beobachtet oder nachgewiesen werden kann. Bevorzugt geschieht dies quantifiziert im Verlauf der Amplifizierung.

Beim ABI-TaqMan-Verfahren wird nach dem Prinzip des Floureszenzenergietransfers gearbeitet. Die Taq-Man-Sonde (ein Oligonukleotid, das am Template dort hybridisiert, bis dahin das Template während der PCR-Reaktion aufgebaut wird) wird am 5- bzw. 3-Ende des Oligonukleotids mit einem Donor- bzw. Akzeptorfarbstoff markiert. Dadurch ist nur die Akzeptorfluoreszenz detektierbar, die Donorfluoreszenz wird durch den Energietransfer zum Akzeptor gelöscht. Nach erfolgreichem Aufbau des Templates während der PCR-Reaktion wird die 5- terminale Base der TacMan-Sonde abverdaut. Dadurch geht der räumlich nahe, fixierte Kontakt zum Akzeptorfarbstoff verloren und das Fluoreszenzsignal steigt. Für die Referenzierung wird zusätzlich noch ein freier Farbstoff, der bei einer anderen Wellenlänge emitiert, der Lösung zugesetzt.

Nachteilig an diesem Verfahren ist, daß die Probe durch die Beigabe von Fluoreszenzfarbstoffen, die für die eigentliche Messung erforderlich sind, verunreinigt wird. Eine Weiterverarbeitung der Probe ist nicht direkt möglich. Dadurch erforderliche Reinigungsschritte bergen das Risiko in sich, daß die Probe durch die fehlgeschlagene Reinigung unbrauchbar wird.

EP-A-0 819769 offenbant ein Verfahren zur Detektion z.B. eines Enzymproduktes nach einer Amplifizierung durch Streulicht-und Fluoreszenz-messungen.

Aufgabe der vorliegenden Erfindung ist es daher ein Verfahren und eine Vorrichtung zur Quantifizierung von DNA und RNA Sequenzen bereitzustellen, die im Online-Betrieb einfacher und effizienter als die Verfahren und Vorrichtungen des Standes der Technik eingesetzt werden können.

Diese Aufgabe wird durch das Verfahren und die Vorrichtung nach den unabhängigen Ansprüchen gelöst. Weitere vorteilhafte Ausgestaltungen sind in den abhängigen Ansprüchen angegeben.

Insbesondere wird die Aufgabe durch ein Verfahren zur Online-Detektion einer Amplifizierung einer DNA- und/oder RNA-Sequenz in einer Probe gelöst, bei der eine Bewertung der Amplifizierung der DNA- und/oder RNA-Sequenz in der Probe aufgrund eines Streulichtssignals der Probe erfolgt. Es war hierbei überraschend gefunden worden, daß die Amplifikation von DNA oder RNA ohne Zusätze online detektiert werden konnten. Hierbei wurde das Streulichtsignal der RNA oder DNA-Moleküle ausgenutzt. Das erfindungsgemäße Verfahren beruht auf der Tatsache, daß die Intensität der Rayleigh-Streuung (Teilchengröße $\ll \lambda$, Wellenlänge des Lichtes) proportional zur Lichtintensität $I_0$, der Molekülgröße $M_C$ und der Konzentration c der Teilchen ist.

$$I \sim I_0\ M_C\ c$$

Durch die Ausnutzung des Streulichtsignals ist es somit nicht mehr nötig, Fluoreszenzfarbstoffe wie FAM, JOE, TAMRA und ROX einzusetzen. Durch eine kontinuierliche Erfassung des Streulichtsignals kann eine quantitative Aussage zur Amplifizierung vorgenommen werden.

Bei einem weiteren bevorzugten erfindungsgemäßen Verfahren wird die Probe durch eine Quelle angeregt, wobei die Quelle eine Lichtquelle, bevorzugt eine Lampe, ein Laser oder eine Leuchtdiode ist. Besonders bevorzugt wird eine Xenonlampe oder ein Helium-Neonlaser eingesetzt. Bei dieser bevorzugten Ausführungsform der vorliegenden Erfindung können die Quellen unterschiedlichster Emissionsspektren eingesetzt werden, da die Information aus dem Streulichtsignal gewonnen wird. Es ist daher nicht wie bei den Verfahren des Standes der Technik notwendig, daß eine spezielle Laserlichtquelle eingesetzt werden muß.

Bei dem weiteren bevorzugten Ausführungsbeispiel der vorliegenden Erfindung ist ein Verfahren vorgesehen, bei dem das Streulichtsignal zur Streulichtintensität korrespondiert. Eine derartige Korrespondenz kann mit Hilfe eines Detektors, besonders bevorzugt einer Pin-Diode, registriert werden. Damit ist es möglich, die Amplifizierung quantitativ zu bestimmen.

Das erfindungsgemäße Verfahren weist aufgrund seiner Einfachheit mehrere Vorteile auf. Es muß kein Primer oder Nukleotid markiert werden und die Probe kann anschließend ohne Reinigungsschritte direkt weiterverarbeitet werden. Die Reaktion über die Streulichtintensität kann online detektiert werden und ermöglicht so eine sicherere, einfachere und kostenbewußtere Möglichkeit der Detektion einer Amplifizierung. Bevorzugt ist es auch möglich, die Amplifizierung durch eine Anfangs- und Endpunktbestimmung zu überprüfen.

Bei einem weiteren bevorzugten Verfahren der vorliegenden Erfindung enthält die Probe Verunreinigungen, insbesondere sind fremde DNA- und/oder RNA-Sequenzen in der Probe vorhanden. Während bei Absorptionsssmessungen die zugesetzten Mononukleotide eine Verfolgung der Amplifikation verhindern, wurde überraschend festgestellt, daß die zugesetzten Mononukleotide eine Streulichtintensitätsmessung nicht beeinflussen. Daher wurde als weiterer Vorteil der vorliegenden Erfindung gefunden, daß das erfindungsgemäße Verfahren eine Amplifizierung einer gewünschten DNA- und/oder RNA-Sequenz in einer Probe auch dann detektieren kann, wenn "fremde" DNA, RNA und/oder Proteine vorhanden sind, da nur die Zunahme der Streulichtintensität gemessen wird. Bei einer Online-Detektion der Amplifizierung können sich somit beispielsweise die Viskosität und/oder andere Eigenschaften der Lösung verändern, ohne daß das erfindungsgemäße Verfahren dadurch beeinflußt würde. Besonders bevorzugt wird das Verfahren daher auch zur Detektion, insbesondere Online-Detektion, bei einer Amplifizierung einer DNA- und/oder RNA-Sequenz in einer im übrigen verunreinigten Probe durchgeführt. Es sind daher Selbstmessungen in DNA- oder RNA-Proben möglich, die aus Zellkulturen entnommen wurden und im Überschuß "fremd" - DNA bzw. RNA enthalten.

Bei einem weiteren bevorzugten Ausführungsbeispiel der vorliegenden Erfindung ist ein Verfahren vorgesehen, bei dem für bekannte Ausgangs- oder Endkonzentrationen von Produkten oder Edukten eine quantitative Bestimmung der Produkte und/oder Edukte erfolgt. Neben der nicht selektiven, qualitativen Aussage, ob eine Amplifikation stattgefunden hat, erlaubt das erfindungsgemäße Verfahren ebenso eine quantitative Bestimmung der Produkte oder Edukte. Bevorzugt wird dafür eine Echtzeit-Detektion (Online) vorgesehen. Für diese Aussage werden bevorzugt die Ausgangs- oder Endkonzentrationen der Produkte/Edukte mitberücksichtigt.

Das erfindungsgemäße Verfahren kann sowohl für temperature cycling Amplifizierung (polymerase chain reaction PCR (RT-PCR), ligase chain reaction LCR, transcriptionbased amplification), als auch für isotherme Amplifizierungen (strand displacement amplification, nucleic acid sequence based amplification NASBA, Qß-replicase system) und weitere Amplifizierungsreaktionen eingesetzt werden.

Die Aufgabe wird weiterhin durch eine Vorrichtung zur Quantifizierung einer Amplifizierung einer DNA- und/oder RNA-Sequenz in einer Probe gelöst, die folgende Komponenten umfaßt: eine Einrichtung zur Anregung der Probe und eine Detektionseinrichtung, wobei durch die Detektionseinrichtung ein Streulichtsignal aus der Probe detektierbar ist. Mit dieser Vorrichtung zur Streulichtmessung wurde überraschend gefunden, daß eine Aussage über die Amplifizierung der DNA- und/ oder RNA-Sequenz in einer Probe möglich ist.

Bei einem weiteren vorteilhaften Ausführungsbeispiel der vorliegenden Erfindung umfaßt die Einrichtung zur Anregung eine Lichtquelle bevorzugt eine Lampe, einen Laser oder besonders bevorzugt eine Leuchtdiode. Bei dieser bevorzugten Ausführungsform der vorliegenden Erfindung können die Quellen unterschiedlichster Emissionsspektren eingesetzt werden, da die Information aus dem Streulichtsignal gewonnen wird. Es ist daher nicht wie bei den Verfahren des Standes der Technik notwendig, daß eine Laserlichtquelle mit spezieller Frequenzbreite eingesetzt werden muß.

Eine weitere bevorzugte Ausführungsform zeichnet sich dadurch aus, daß die Detektionseinrichtung einen Photomultiplier (PMT) und/ oder eine CCD-Kamera und/ oder eine Diode umfaßt, insbesondere eine Avalanchephotodiode (APD) und/ oder mindestens eine PIN-Diode (16). Mit einem Photomultiplier und/ oder einer CCD-Kamera und/ oder einer Diode ist die Erfassung des Streulichtsignals möglich. Bevorzugt wird eine Kombination von verschieden geschalteten PIN-Dioden verwendet, wodurch der speziellen Meßsituation der einzelnen Detektionseinrichtungen Rechnung getragen werden kann. So ist es möglich, daß durch den Einsatz von Filtern Streulichtsignale vorbestimmter Frequenzbereiche bevorzugt detektiert werden. Es ist ebenfalls möglich, daß die Signale von verschiedenen PIN-Dioden erfaßt werden und erst eine Kombination dieser verschiedenen Signale das Endsignal definiert. Als Detektionseinrichtung kann ebenfalls an eine Bilderfassungseinrichtung gedacht werden, bevorzugt an eine CCD-Kamera. Vorteilhafterweise werden bevorzugt ein PMT und/ oder ein ADP eingesetzt, wenn geringere Substanzmengen detektiert werden sollen, da diese sehr empfindlich sind.

Eine weitere bevorzugte Vorrichtung der vorliegenden Erfindung umfaßt weiter eine Abscanneinrichtung. Mit dieser Abscanneinrichtung ist es möglich, daß ein spezielles Streulichtsignal aus der Probe an die Detektionseinrichtung übergeben werden kann. Bei diesem bevorzugten Ausführungsbeispiel der Erfindung ist somit eine gezielte Übergabe der Streulichtsignale der speziellen Probe an einen speziellen Detektor möglich.

Bei einer weiteren bevorzugten Vorrichtung der Erfindung sind mehrere Probenträger, bevorzugt Microtiterplatten oder Kapillaren, vorgesehen. Damit ist es möglich, mehrere Proben in einem Arbeitsgang zu beobachten und bevorzugt abzuscannen. Es sind dadurch effizientere Beobachtungs- und Detektionsmöglichkeiten gegeben. Es können außerdem Serien an Proben simultan beobachtet werden und damit zusammengehörige Messungen auf einmal abgearbeitet werden.

Eine weitere bevorzugte erfindungsgemäße Vorrichtung weist Probenträger auf, die mit der Abscanneinrichtung abgescannt werden können. Dadurch ist es möglich, daß die Probenträger beispielsweise in x-y-Richtung abgescannt werden können. Dabei befinden sich die Proben in den Probeträgern bevorzugt in einer Ebene und werden nacheinander durch die Abscanneinrichtung angefahren und gemessen. Ebenso ist es möglich, daß die Probenträger als solche beweglich ausgebildet sind und so bewegt werden, daß eine Probe nach der anderen durch die Abscanneinrichtung erfaßt werden kann. Außerdem ist es bevorzugt, wenn sowohl die Probenträger als auch die Abscanneinrichtung beweglich ausgebildet sind, so daß ein Austausch der Probenträger und ein Verschwenken der Abscanneinrichtung zu einer optimalen Ausnutzung der Rüst- und Beschickungszeiten der erfindungsgemäßen Vorrichtung führt. So kann der feststehende Probenträger durch die bewegliche Abscanneinrichtung abgescannt werden und danach die Abscanneinrichtung zu einem weiteren Feld an Probenträgern bewegt werden, während die ersten Probenträger weiterbehandelt oder ausgetauscht werden.

Eine bevorzugte weitere erfindungsgemäße Vorrichtung sieht eine Abscanneinrichtung vor, die einen bevorzugt beweglichen Spiegel umfaßt, über den ein Abtaststrahl der Abscanneinrichtung gerichtet werden kann. Auf diese Weise ist es möglich, daß die Abscanneinrichtung an sich fixiert ist und nicht bewegt werden muß, aber auch die Probenträger nicht bewegt werden müssen. Es reicht in diesem bevorzugten Ausführungsbeispiel aus, daß der Spiegel bewegt wird und dadurch die entsprechenden Streulichtsignale der einzelnen Proben in den Probenträgern an die Detektionseinrichtung weitergegeben werden. Hierbei ist es bevorzugt vorgesehen, daß die Probenträger in einer vorbestimmten Reihenfolge besonders bevorzugt zyklisch abgescannt werden und dadurch mittels der bekannten Position des Spiegels für jede Probe eine quasi-kontinuierliche Erfassung des Streulichtsignals möglich ist. So kann beispielsweise zu einer Zeit t1 ein Probenträger P1 erfaßt werden, zu einer Zeit t1 + T ein Probenträger P2, etc. bis zum Zeitpunkt t1 + NT wieder der Probenträger P1 erfaßt wird, wobei N die Anzahl der zu erfassenden Probenträger P und T die Zeit der Messung und der Erfassung der folgenden Probe darstellt. Damit ergibt sich für die spezielle Probe x in dem Probenträger Px eine quasi-kontinuierliche Erfassung der Streulichtsignale und damit des Verlaufs der Amplifizierung einer DNA- und/ oder RNA-Sequenz in einer Probe x mittels Interpolation der Meßwerte der Probe x zu den Zeiten

$$\text{tx, tx + NT, tx + 2NT, ..., tx + iNT etc.}$$

Eine weitere bevorzugte Ausführungsform der vorliegenden Erfindung stellt eine Vorrichtung dar, bei der die Einrichtung zur Anregung so ausgestaltet ist, daß die Probenträger bzw. Proben großflächig anregbar sind und die zu den einzelnen Probenträgern korrespondierenden Streulichtsignale durch die Detektionseinrichtung einzeln erfaßbar sind. Hierdurch ist es möglich, daß eine großflächige Probe bzw. Probenfelder bzw. auf großer Fläche verteilte Probenträger simultan erfaßt und ausgewertet werden können. Es genügt dabei die stetige diffuse Anregung des Probenfeldes, da für jede Probe bzw. jeden Ausschnitt des Probenfeldes die relative Streulichtintensität erfaßt wird, die von dem absoluten Streulichtsignal an dieser Stelle unabhängig ist.

Bei einer weiteren bevorzugten Ausführungsform der vorliegenden Erfindung ist eine Vorrichtung vorgesehen, bei der die Detektionseinrichtung mindestens zwei einzelne Detektoren umfaßt, wobei durch die einzelnen Detektoren unterschiedliche Streulichtsignale erfaßbar sind. Auf diese Weise ist es möglich, daß mehrere Proben simultan und nicht leicht zeitversetzt erfaßt werden können. Dies ist insbesondere dann vorteilhaft, wenn eine genau parallele Auswertung der Proben gewünscht ist. Besonders bevorzugt werden die einzelnen Detektoren über Lichtwellenleiter mit den Probeträgern bzw. Proben verbunden, so daß mit Hilfe eines Lichtwellenleiterbündels auch Profile von Probenfeldern erfaßt werden können. Bevorzugt ist ebenfalls daran zu denken, daß ein großflächiges Probenfeld mittels einer CCD-Kamera aufgezeichnet wird und die Detektion der Streulichtsignale an den einzelnen Stellen mittels der Auswertung des Bildes, bevorzugt über eine Steuereinrichtung durchgeführt wird, besonders bevorzugt über einen Rechner oder einen Computer, besonders bevorzugt über ein Bildverarbeitungssystem.

Bevorzugt ist weiter eine Steuereinheit vorgesehen ist, zu der Signale übertragbar sind, die zu den erfaßten Streulichtsignalen korrespondieren und diese von der Steuereinheit auswertbar sind. Durch diese Steuereinheit können die einzelnen Meßwerte in den entsprechenden Matrizen für die einzelnen Proben verarbeitet werden und an eine Speichereinheit zur Speicherung weitergegeben werden. Darüber hinaus kann über die Steuereinheit die Steuerung eines Abtastscanners vorgenommen werden und es können die Detektoren eingestellt werden bezüglich beispielsweise der der Empfindlichkeit und der Ausrichtung auf die Proben hin.

Daneben besteht eine vorteilhafte Anwendung der vorliegenden Erfindung darin, daß eine Vorrichtung zur Streulichtmessung zur Quantifizierung einer Amplifizierung einer DNAund/ oder RNA-Sequenz in einer Probe vorgenommen wird.

Im folgenden sollen weitere vorteilhafte Ausgestaltungen der Erfindung an Hand der Zeichnung erläutert werden. Hierbei zeigt

Fig. 1    ein Diagramm einer Streulichtintensitätsmessung nach einem Ausführungsbeispiel eines erfindungsgemäßen Verfahrens bei einer gegebenen Konzentration (a) und ein Diagramm einer Streulichtintensitätsmessung nach einem Ausführunsgbeispiel des erfindungsgemäßen Verfahrens aus (a) bei zwei verdünnten Konzentration und einer Blindprobe (b);

Fig. 2    ein Diagramm, das eine Vergleichsmessung zwischen einer Floureszenzmessung nach TaqMan nach dem Stand der Technik und der erfindungsgemäßen Streulichtmessung umfaßt (a) und ein Diagramm, das eine Vergleichsmessung zwischen einer Floureszenzmessung durch Interkalationsfarbstoff nach dem Stand der Technik und der erfindungsgemäßen Streulichtmessung zeigt (b);

Fig. 3    einen schematischen Aufbau eines Ausführungsbeispiels der erfindungsgemäßen Vorrichtung mit einer Probe;

Fig. 4    einen schematischen Aufbau eines Ausführungsbeispiels der erfindungsgemäßen Vorrichtung mit einer Vielzahl von Proben bzw. Probenträgern und einer Abscanneinrichtung; und

Fig. 5    einen schematischen Aufbau eines Ausführungsbeispiels der erfindungsgemäßen Vorrichtung mit einer Vielzahl von Proben bzw. Probenträgern und Lichtwellenleitern

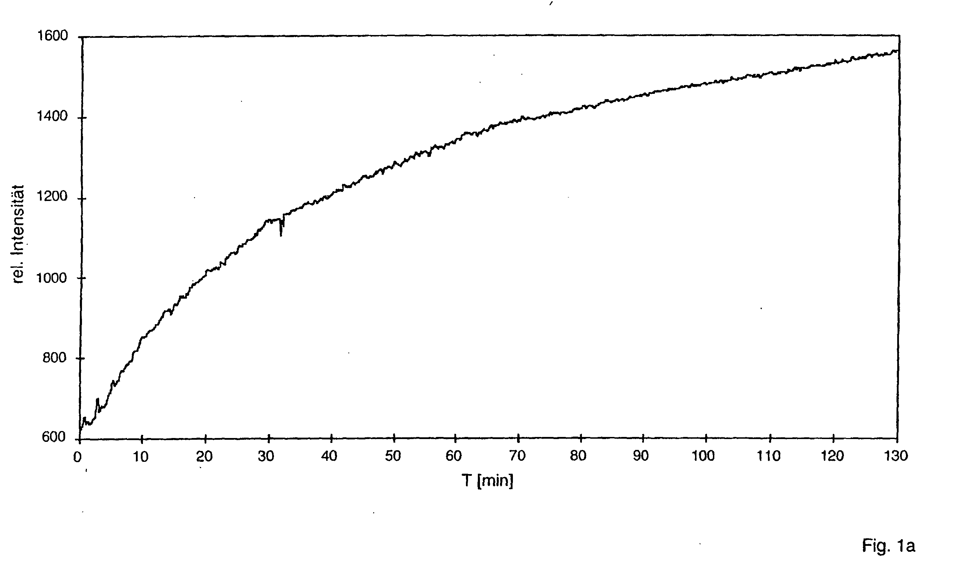

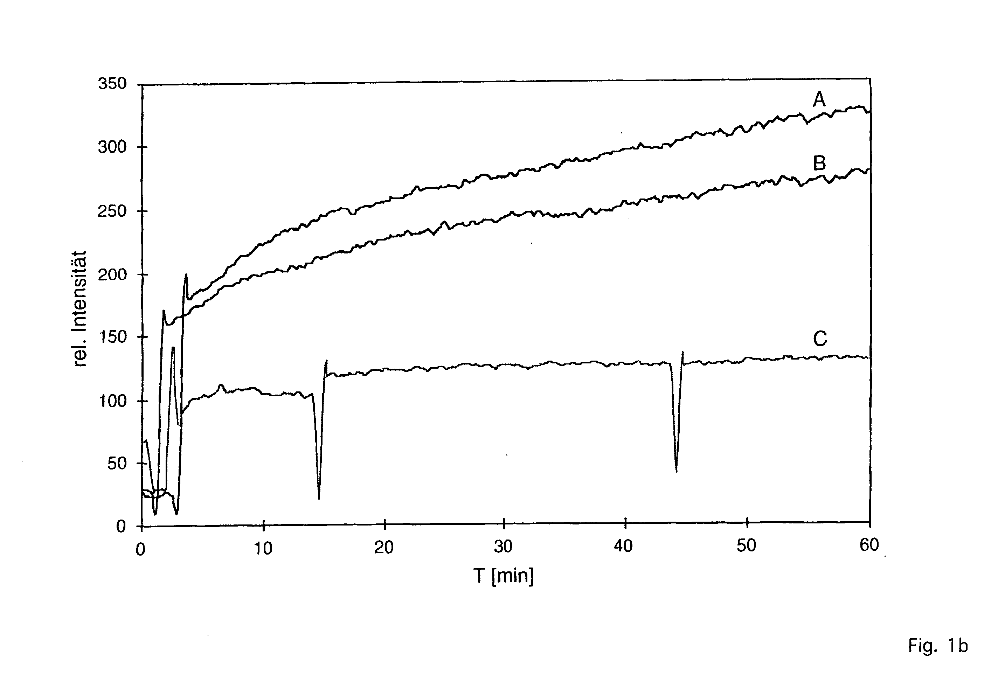

Fig. 1 a zeigt ein Diagramm einer Streulichtintensitätsmessung nach einem Ausführunsgbeispiel eines erfindungsgemäßen Verfahrens bei einer gegebenen Konzentration. Deutlich zu erkennen ist der Anstieg der relativen Streulichtintensität mit der Zeit. Dieser Verlauf repräsentiert die voranschreitende Amplifizierung innerhalb der Probe.

In Fig 1 b ist ein Diagramm einer Streulichtintensitätsmessung nach einem Ausführunsgbeispiel des erfindungsgemäßen Verfahrens aus Fig. 1 a bei zwei verdünnten Konzentration (Kurven A und B) dargestellt. Darüber hinaus ist der Verlauf einer Blindprobe (Kurve C) dargestellt. Dadurch wird erkenntlich, daß mit einer Streulichtintensitätsmessung ebenfalls eine qualitative Aussage aufgrund der relativen Streulichtintensität getroffen werden kann. Im Falle der Blindprobe (Kurve C) ist kein Anstieg der relativen Streulichtintensität zu verzeichnen. Am Anfang der Messung wurden im Falle A und B Enzyme zugegeben, die die Amplifizierung ausgelöst haben. Eine Zugabe von Mononukleotidtriphosphat unterblieb in der Blindprobe.

Es handelt sich in beiden Fällen in Fig. 1 a und Fig. 1 b um eine AmpliScribe ™ SP6-Reaktion (Epicentre Technologies) - das ist eine kommerziell erhältliche Amplifizierung von RNA durch Transkription - bei einer Reaktionstemperatur von 39° C .

| Komponenten | Volumen | Finalkonzentration | | |
|---|---|---|---|---|
| | Kurve A/ B/ C | Kurve A | Kurve B | Kurve C |
| ATP (100 mM) | 2 / 2 / 0 μl | 2,8 mM | 2,3 mM | 0 mM |
| CTP (100 mM) | 2 / 2 / 0 μl | 2,8 mM | 2,3 mM | 0 mM |
| GTP (100 mM) | 2 / 2 / 0 μl | 2,8 mM | 2,3 mM | 0 mM |
| UTP (100 mM) | 2 / 2 / 0 μl | 2,8 mM | 2,3 mM | 0 mM |
| DTT (100 mM) | 4 μl | 5,6 mM | 4,6 mM | 5 mM |
| AmpliScribe SP6 Enzymlösung | 4 μl | | | |
| Wasser | 47 / 63 / 63 μl | | | |
| 10x SP6 Reaktionspuffer | 7 μl | 1x | 0,8x | 0,9x |
| Kontroll Template DNA (0,5 μg/μl) | 2 μl | 1 μg | 1 μg | 1 μg |
| **Totalvolumen** | **72 / 88 / 89 μl** | | | |

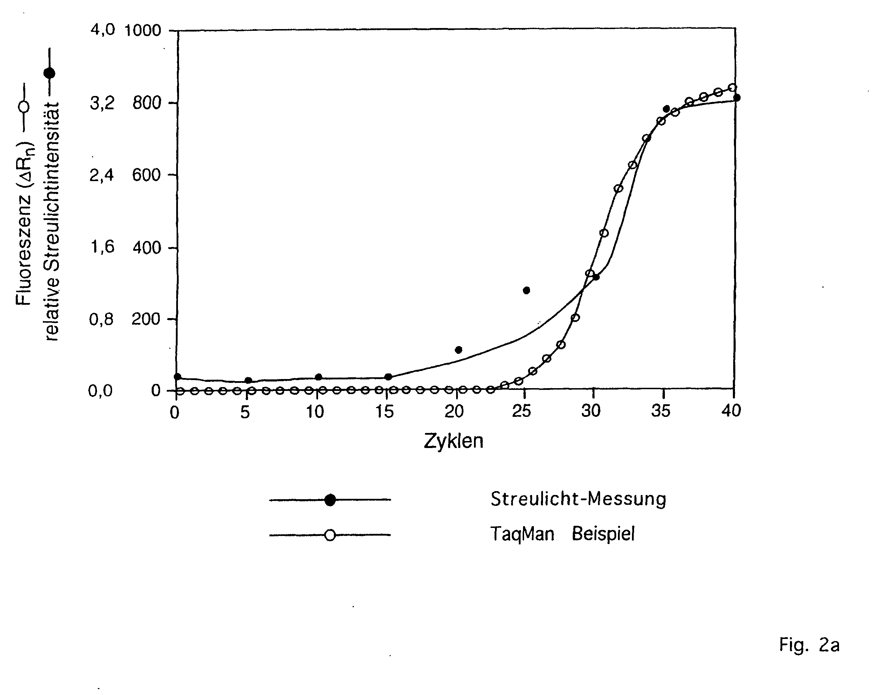

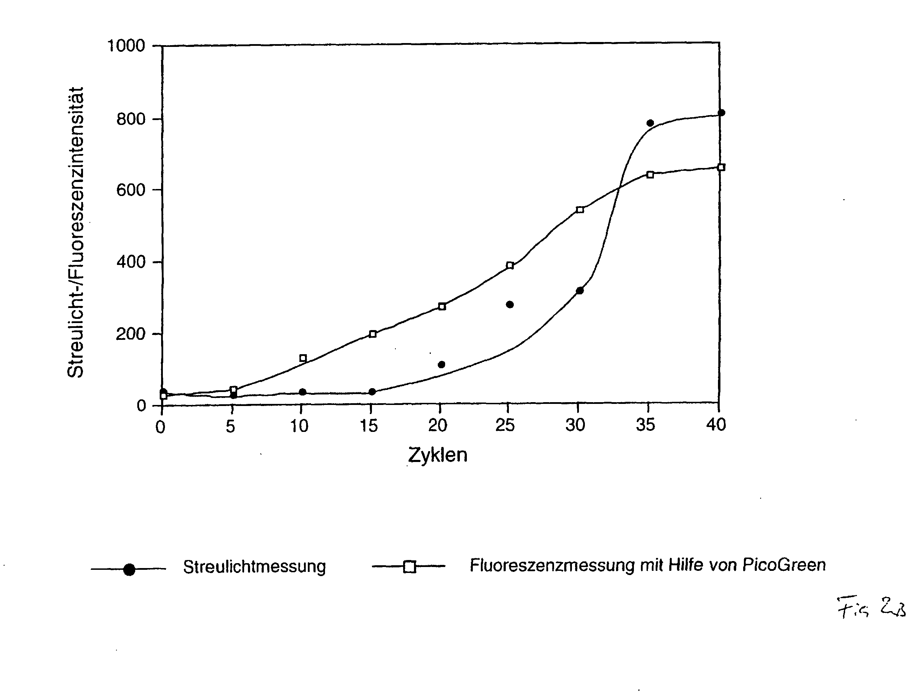

In Fig. 2 a ist ein Diagramm, das eine Vergleichsmessung zwischen einer Floureszenzmessung nach dem Stand der Technik und der erfindungsgemäßen Streulichtmessung umfaßt, dargestellt. Die Meßpunkte der erfindungsgemäßen Streulichtmessung wurde mit vollen Punkten dargestellt, während die Floureszenzmessung nach TaqMan mit Kreisen dargestellt ist. Es ist gut erkennbar, daß durch beide Methoden die Amplifizierung nachgewiesen werden kann.

Die Versuchsbedingungen für die erfindungsgemäße Streulichtmessung war wie folgt:

| Komponenten | Vol./Reaktion | Finalkonz./Reaktion |
|---|---|---|
| ATP (10 mM) | 1 μl | 200 μM |
| CTP (10 mM) | 1 μl | 200 μM |
| GTP (10 mM) | 1 μl | 200 μM |
| TP (10 mM) | 1 μl | 200 μM |
| Primer A | variabel | 0,1 μM |
| Primer B | variabel | 0,1 μM |

(fortgesetzt)

| Komponenten | Vol./Reaktion | Finalkonz./Reaktion |
|---|---|---|
| aq DNA Polymerase (5U/µl) | 0,5 µl | 2,5 U |

| Komponenten | Vol./Reaktion | Finalkonz./Reaktion |
|---|---|---|
| Wasser | variabel | |
| 10x PCR Puffer | 5 µl | 1x |
| Template DNA | variabel | ca. 0,25 µg/Reaktion |

| Totalvolumen | | | **50 µl** | |
|---|---|---|---|---|
| Cyclingbedingung: | 95 °C | 120 s | | |
| | 95 °C | 20 s | | |
| | 60°C | 30 s | | |
| | 72° C | 60 s | 40 x Zyklen | |

Nach jeweils fünf Zyklen wird eine Probe beim Ende des 72° C-Schrittes für die Streulichtmessung entnommen und mit 50 µl Wasser verdünnt.

In Fig. 2 b ist ein Diagramm dargestellt, das eine Vergleichsmessung zwischen einer Floureszenzmessung durch Interkalationsfarbstoff nach dem Stand der Technik und der erfindungsgemäßen Streulichtmessung zeigt. Diese Vergleichsmessung wurde mit dem Interkalator PicoGreen ausgeführt:

Es wurden identische Proben wie bei der Streulichtintensitätsmessung nach Fig. 2 a verwendet, jedoch nur 2 µl der Reaktionslösung, verdünnt mit 60 µl Wasser und 20 µl PicoGreen (1:20 Verdünnung) Lösung. Eine Anregung erfolgte bei 480 nm und die Detektion bei 525 nm. Es ist deutlich zu sehen, daß auch hier der Nachweis erbracht wird, daß die PCR-Reaktion geklappt hat.

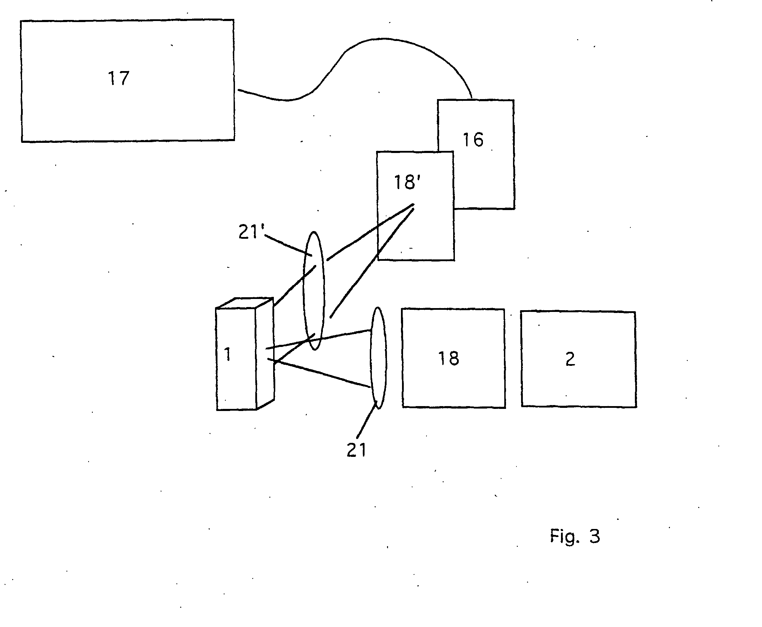

In Fig. 3 ist ein schematischer Aufbau eines Ausführungsbeispiels der erfindungsgemäßen Vorrichtung mit einer Probe dargestellt. Die Probe 1 wird mittels einer Quelle 2 angeregt. Das von der Quelle emittierte Licht wird durch Monochromatoren 18 geleitet und mittels einer Linse 21 auf die Probe fokussiert. Das Streulicht wird mittels der Linse 21' über den Monochromator 18' an die PIN-Diode 16, die als Detektionseinrichtung eingesetzt wird, weitergegeben. An die Detektionseinrichtung ist eine Steuereinheit 17 angeschlossen, die die Signale auswertet und aufzeichnen läßt.

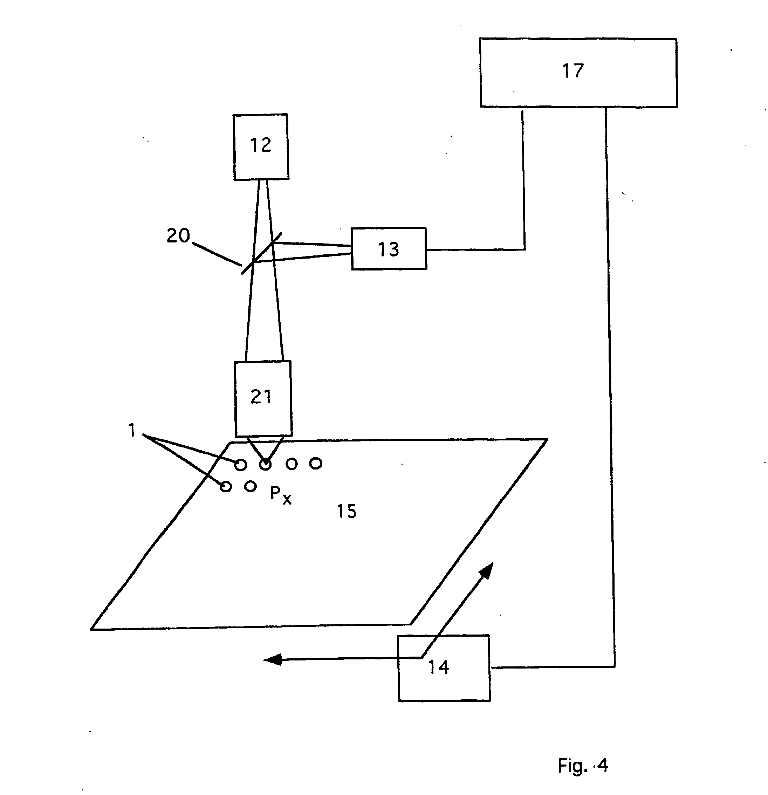

In Fig. 4 ist ein schematischer Aufbau eines Ausführungsbeispiels der erfindungsgemäßen Vorrichtung mit einer Vielzahl von Proben 1 bzw. Probenträgern 15 und einer Abscanneinrichtung dargestellt. Probenträger 15 sind in einem Probenfeld 1 angeordnet. Eine Anregungseinrichtung, hier ein Anregungslaser, emittiert Licht und regt über eine Linse bzw. Mikroskopobjektiv 21 eine Probe Px an. Das gestreute Licht wird über die Linse 21 und die Glasscheibe 4 an die Detektionseinrichtung 13 weitergegeben und dort erfaßt. Die Detektionseinrichtung ist an einer Steuereinheit 17 angeschlossen. Diese Steuereinheit steuert eine Abscanneinrichtung 14, die das Probenfeld 1 bewegen kann.

Im Betrieb gibt die Steuereinrichtung einen Steuerimpuls an die Abscanneinrichtung und veranlaßt diese, eine spezielle Probe in den Fokusbereich der Linse 21 zu bewegen. Danach wird eine Messung vorgenommen, der Meßwert erfaßt und gespeichert und daraufhin durch ein weiteres Steuersignal der Steuereinheit 17 die Abscanneinrichtung angesteuert, die eine nächste Probe in den Fokusbereich der Linse 21 fährt, wo jetzt ebenfalls eine Messung stattfindet. So können die Proben nacheinander und bevorzugt zyklisch erfaßt und gemessen werden.

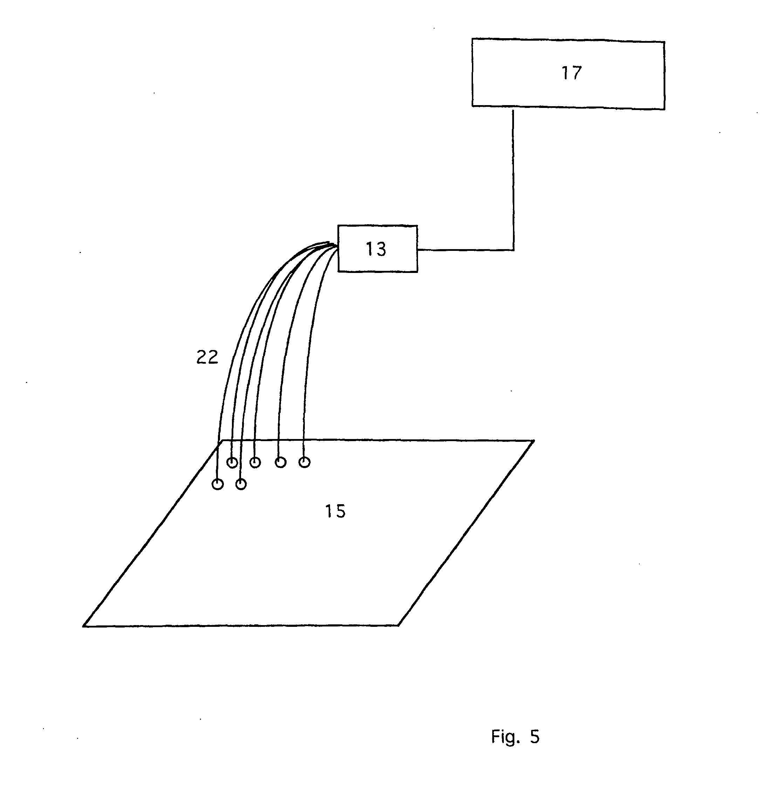

In Fig. 5 ist ein schematischer Aufbau eines Ausführungsbeispiels der erfindungsgemäßen Vorrichtung mit einer Vielzahl von Proben bzw. Probenträgern und Lichtwellenleitern dargestellt. Hier ist die Detektionseinrichtung 13 über Lichtwellenleiter 22 mit den Proben 1 verbunden. Über die Lichtwellenleiter wird sowohl das Anregungslicht übertragen als auch das Streulicht erfaßt. Die Detektionseinrichtung gibt die erfaßten Streulichtsignale an die Steuereinrichtung 17 weiter, wo sie verarbeitet und gegebenenfalls zwischengespeichert werden.

Es wurde somit eine Vorrichtung und ein Verfahren zur Detektion, insbesondere Online-Detektion, einer Amplifizierung einer DNA- und/ oder RNA-Sequenz in einer Probe bereitgestellt, wobei eine Bewertung der Amplifizierung der DNA- und/ oder RNA-Sequenz in der Probe aufgrund eines Streulichtsignals der Probe erfolgt. Dieses Verfahren und diese Vorrichtung zur Quantifizierung von DNA und RNA Sequenzen können im Online-Betrieb einfacher und effizienter als die Verfahren und Vorrichtungen des Standes der Technik eingesetzt werden.

BEZUGSZEICHENLISTE

1     Probe

2     Quelle, insbesondere Lichtquelle

12   Einrichtung zur Anregung

13   Detektionseinrichtung

14   Abscanneinrichtung

15   Probenträger

16   PIN-Diode

17   Steuereinheit

18   Monochromator

19   Spiegel

20   Glasplatte

21   Linse

22   Lichtwellenleiter

**Patentansprüche**

1. Verfahren zur Detektion, insbesondere Online-Detektion, einer Amplifizierung einer DNA- und/ oder RNA-Sequenz in einer Probe (1)
**dadurch gekennzeichnet, daß**
eine Bewertung der Amplifizierung der DNA- und/oder RNA-Sequenz in der Probe (1) aufgrund eines Streulichtsignals der Probe (1) erfolgt.

2. Verfahren nach Anspruch 1 **dadurch gekennzeichnet, daß** die Probe (1) durch eine Quelle (2) angeregt wird, wobei die Quelle (2) eine Lichtquelle, insbesondere eine Lampe, ein Laser oder eine Leuchtdiode ist.

3. Verfahren nach einem der vorhergehenden Ansprüche **dadurch gekennzeichnet, daß** das Streulichtsignal zur Streulichtintensität korrespondiert.

4. Verfahren nach einem der vorhergehenden Ansprüche **dadurch gekennzeichnet, daß** die Probe Verunreinigungen enthält, insbesondere daß fremde DNA- und/oder RNA-Sequenzen in der Probe vorhanden sind.

5. Verfahren nach einem der vorhergehenden Ansprüche **dadurch gekennzeichnet, daß** für bekannte Ausgangs- oder Endkonzentrationen von Produkten oder Edukten eine quantitative Bestimmung der Produkte und/oder Edukte erfolgt.

6. Vorrichtung umfassend
eine Einrichtung (12) zur Anregung einer Probe (1), Mittel zur Quantifizierung einer Amplifizierung einer DNA- und/ oder RNA-Sequenz in der Probe (1) gemäß einem der Verfahren nach einem der vorhergehenden Ansprüche, des eine Detektionseinrichtung (13) umfaßt, durch die ein Streulichtsignal aus der Probe (1) detektierbar ist.

7. Vorrichtung nach Anspruch 6 **dadurch gekennzeichnet, daß** die Einrichtung (12) zur Anregung eine Lichtquelle umfaßt, insbesondere eine Lampe, einen Laser oder eine Leuchtdiode umfaßt.

**8.** Vorrichtung nach einem der Ansprüche 6 oder 7 **dadurch gekennzeichnet, daß** die Detektionseinrichtung (13) einen Photomultiplier und/ oder eine CCD-Kamera und/ oder eine Diode umfaßt, insbesondere eine Avalanchephotodiode und/ oder mindestens eine PIN-Diode (16).

**9.** Vorrichtung nach einem der Ansprüche 6 bis 8 **dadurch gekennzeichnet, daß** weiter eine Abscanneinrichtung (14) vorgesehen ist.

**10.** Vorrichtung nach einem der Ansprüche 6 bis 9 **dadurch gekennzeichnet, daß** mehrere Probenträger (15), insbesondere Microtiterplatten oder Kapillaren, vorgesehen sind.

**11.** Vorrichtung nach einem der Ansprüche 9 bis 10 **dadurch gekennzeichnet, daß** die Probenträger (15) mit der Abscanneinrichtung (14) abgescannt werden können.

**12.** Vorrichtung nach einem der Ansprüche 9 bis 11 **dadurch gekennzeichnet, daß** die Abscanneinrichtung (14) einen bevorzugt beweglichen Spiegel (19) umfaßt, über den ein Abtaststrahl der Abscanneinrichtung (14) gerichtet werden kann.

**13.** Vorrichtung nach einem der Ansprüche 6 bis 12 **dadurch gekennzeichnet, daß** die Einrichtung (12) zur Anregung so ausgestaltet ist, daß die Probenträger (15) großflächig anregbar sind und die zu den einzelnen Probenträgern (15) korrespondierenden Streulichtsignale durch die Detektionseinrichtung (13) einzeln erfaßbar sind.

**14.** Vorrichtung nach einem der Ansprüche 6 bis 13 **dadurch gekennzeichnet, daß** die Detektionseinrichtung (13) mindestens zwei einzelne Detektoren umfaßt, wobei durch die einzelnen Detektoren unterschiedliche Streulichtsignale erfaßbar sind.

**15.** Vorrichtung nach einem der Ansprüche 6 bis 14 **dadurch gekennzeichnet, daß** weiter eine Steuereinheit (17) vorgesehen ist, zu der Signale übertragbar sind, die zu den erfaßten Streulichtsignalen korrespondieren und diese von der Steuereinheit auswertbar sind.

**16.** Verwendung einer Vorrichtung zur Streulichtmessung zur Quantifizierung einer Amplifizierung einer DNA- und/ oder RNA-Sequenz in einer Probe (1) gemäß einem der Verfahren nach einem der Ansprüche 1 bis 5.

## Claims

**1.** A method for the detection, especially online detection, of the amplification of a DNA and / or RNA sequence in a sample (1), **characterized in that** the amplification of the DNA and / or RNA sequence in the sample (1) is evaluated based on a scattered light signal of the sample (1).

**2.** The method according to claim 1, **characterized in that** the sample (1) is excited by a source (2), whereby the source (2) is a light source, especially a lamp, laser or LED.

**3.** The method according to one of the preceding claims, **characterized in that** the scattered light signal corresponds to the scattered light intensity.

**4.** The method according to one of the preceding claims, **characterized in that** the sample contains impurities, especially foreign DNA and/or RNA sequences.

**5.** The method according to one of the preceding claims, **characterized in that** the quantities of products and / or educts are determined for known initial or end concentrations of products and/or educts.

**6.** A device comprising a means (12) for exciting a sample (1), means to quantify an amplification of a DNA and / or RNA sequence in the sample (1) according to one of the methods according to one of the preceding claims comprising a detector (13) by which a scattered light signal from the sample (1) can be detected.

**7.** The device according to claim 6, **characterized in that** the excitation device (12) is a light source, especially a lamp, laser or LED.

8. The device according to one of claims 6 or 7, **characterized in that** the detector (13) is a photomultiplier and / or a CCD camera and/or a diode, especially an avalanche photodiode and / or at least one PIN diode (16).

9. The device according to one of claims 6 to 8, **characterized in that** a scanner (14) is also provided.

10. The device according to one of claims 6 to 9, **characterized in that** several sample carriers (15) are provided, especially microtiter plates or capillaries.

11. The device according to one of claims 9 to 10, **characterized in that** the sample carriers (15) can be scanned with the scanner (14).

12. The device according to one of claims 9 to 11, **characterized in that** the scanner (14) comprises a mirror (19) that preferably moves and can be used to direct a scanning beam from the scanner (14).

13. The device according to one of claims 6 to 12, **characterized in that** the excitation device (12) is designed so that large areas of the sample carrier (15) can be excited, and scattered light signals that correspond to the individual sample carriers (15) can be individually detected by the detector (13).

14. The device according to one of claims 6 to 13, **characterized in that** the detection device (13) has at least two individual detectors that can detect different scattered light signals.

15. The device according to one of claims 6 to 14, **characterized in that** a controller (17) is also provided that can be sent and can evaluate signals which correspond to the detected scattered light signals.

16. The use of a device to measure scattered light to quantify an amplification of a DNA and / or RNA sequence in a sample (1) according to a method according to one of claims 1 to 5.

## Revendications

1. Procédé pour la détection, en particulier la détection en ligne, d'une amplification d'une séquence d'ADN et/ou d'ARN dans un échantillon (1), **caractérisé en ce que** l'on effectue une évaluation de l'amplification de la séquence d'ADN et/ou d'ARN dans l'échantillon (1) sur base d'un signal de lumière diffusée par l'échantillon (1).

2. Procédé selon la revendication 1, **caractérisé en ce que** l'échantillon (1) est excité par une source (2), la source (2) étant une source de lumière, en particulier une lampe, un laser ou une diode luminescente.

3. Procédé selon l'une des revendications précédentes, **caractérisé en ce que** le signal de lumière diffusée correspond à l'intensité de la lumière diffusée.

4. Procédé selon l'une des revendications précédentes, **caractérisé en ce que** l'échantillon contient des impuretés, en particulier **en ce que** des séquences d'ADN et/ou d'ARN d'origine étrangère sont présentes dans l'échantillon.

5. Procédé selon l'une des revendications précédentes, **caractérisé en ce qu'**au cas où les concentrations initiales ou finales des produits ou des réactifs sont connues, on effectue une détermination quantitative des produits et/ ou des réactifs.

6. Dispositif comprenant un dispositif (12) pour l'excitation d'un échantillon (1), des moyens de quantification de l'amplification d'une séquence d'ADN et/ou d'ARN dans l'échantillon (1) par l'un des procédés selon l'une des revendications précédentes, qui comprend un dispositif de détection (13) par lequel un signal de lumière diffusée par l'échantillon (1) peut être détecté.

7. Dispositif selon la revendication 6, **caractérisé en ce que** le dispositif (12) d'excitation 10 comprend une source de lumière, en particulier une lampe, un laser ou une diode luminescente.

8. Dispositif selon l'une des revendications 6 ou 7, **caractérisé en ce que** le dispositif de détection (13) comprend un photomultiplicateur et/ou une camera CCD et/ou une diode, en particulier une photodiode à avalanche et/ou au moins une diode PIN (16).

9. Dispositif selon l'une des revendications 6 à 8, **caractérisé en ce qu'**un dispositif de balayage (14) y est en outre prévu.

10. Dispositif selon l'une des revendications 6 à 9, **caractérisé en ce que** plusieurs porte-échantillons (15), en particulier des plaques de microtitration ou des capillaires, sont prévus.

11. Dispositif selon l'une des revendications 9 a 10, **caractérisé en ce que** les porte-échantillons (15) peuvent être balayés par le dispositif de balayage (14).

12. Dispositif selon l'une des revendications 9 à 11, **caractérisé en ce que** le dispositif de balayage (14) comprend un miroir (19), de préférence mobile, par lequel un faisceau explorateur du dispositif de balayage (14) peut être orienté.

13. Dispositif selon l'une des revendications 6 à 12, **caractérisé en ce que** le dispositif (12) d'excitation est configuré de telle sorte que les porte-échantillons (15) peuvent être excités sur une grande surface et **en ce que** les signaux de lumière diffusée qui correspondent aux porte-échantillons (15) individuels peuvent être détectés individuellement par le dispositif de détection (13).

14. Dispositif selon l'une des revendications 6 à 13, **caractérisé en ce que** le dispositif de détection (13) comprend au moins deux détecteurs individuels, différents signaux de lumière diffusée pouvant être détectés par les détecteurs individuels.

15. Dispositif selon l'une des revendications 6 à 14, **caractérisé en ce qu'**en outre y est prévue une unité de commande (17) a laquelle des signaux qui correspondent aux signaux de lumière diffusée détectés peuvent être transmis, ces signaux pouvant être évalués par l'unité de commande.

16. Utilisation d'un dispositif de mesure de lumière diffusée pour la quantification d'une amplification d'une séquence d'ADN et/ou d'ARN dans un échantillon (1) par l'un des procédés selon l'une des revendications 1 à 5.

Fig. 1a

Fig. 1b

Fig. 2a

Streulichtmessung          Fluoreszenzmessung mit Hilfe von PicoGreen

Fig 2B

Fig. 3

Fig. 4

Fig. 5